(19) Europäisches Patentamt
European Patent Office
Office européen des brevets

(11) **EP 3 130 375 A1**

(12) **EUROPEAN PATENT APPLICATION**
published in accordance with Art. 153(4) EPC

(43) Date of publication:
**15.02.2017 Bulletin 2017/07**

(51) Int Cl.:
**A61N 5/10** (2006.01)

(21) Application number: **14888989.2**

(22) Date of filing: **07.04.2014**

(86) International application number:
**PCT/JP2014/060089**

(87) International publication number:
**WO 2015/155819 (15.10.2015 Gazette 2015/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Mitsubishi Electric Corporation**
**Chiyoda-ku**
**Tokyo 100-8310 (JP)**

(72) Inventors:
• **PU Yuehu**
  **Tokyo 100-8310 (JP)**

• **SAKAMOTO Yusuke**
  **Tokyo 100-8310 (JP)**
• **YAMADA Yukiko**
  **Tokyo 100-8310 (JP)**
• **IKEDA Masahiro**
  **Tokyo 100-8310 (JP)**
• **FUJI Hideki**
  **Tokyo 100-8310 (JP)**

(74) Representative: **Sajda, Wolf E. et al**
**Meissner Bolte Patentanwälte**
**Rechtsanwälte Partnerschaft mbB**
**Postfach 86 06 24**
**81633 München (DE)**

(54) **PARTICLE BEAM TREATMENT-PLANNING APPARATUS AND METHOD FOR SIMULATING PARTICLE BEAM IRRADIATION**

(57)    A sub beam approximation step in which a particle beam is approximated by a collection of a plurality of sub beams having Gaussian distribution and a sub beam dose distribution calculation step in which by simulating the state in which each sub beam of a plurality of sub beams is deflected by a scanning device and travelled, each sub beam dose distribution which is formed inside a patient by each sub beam is calculated, and a dose distribution which is formed inside a patient by the particle beam is obtained by adding the each sub beam dose distribution which is calculated are comprised.

FIG. 1

**Description**

Technical Field

This invention relates to a particle beam treatment-planning apparatus which determines irradiation parameters in a particle beam therapy system for treating cancer, etc. by irradiating a particle beam to an affected part.

Background Art

A particle beam treatment is a method for treating a tumor in which charged particles such as protons or carbon ions are accelerated to the degree of nucleon hundred mega electron volt by using equipment such as an accelerator, and a particle beam which is a bundle of charged particles which are accelerated is irradiated to a patient so as to give a dose to a tumor in a body.

In actual irradiation, it is important to form a dose distribution with respect to a tumor which is proximate as much as possible to a dose distribution which is instructed by a doctor. A dose distribution which is instructed by a doctor is called a target dose distribution. In many cases, a target dose distribution is a dose distribution in which a dose is uniform in a tumor and a dose is made to be as low as possible outside a tumor.

Generally, in a case where a particle beam which is accelerated by an accelerator is irradiated to an object (including a human body), a three dimensional dose distribution in an object has a dose maximum peak at a certain point. This dose maximum peak is called a Bragg peak. Further, in a case where one point of dose maximum peak is provided in three dimensional space, a position of the peak is defined as an "irradiation position" of the particle beam. In order to form a three-dimensional target dose distribution by using a particle beam having the above-mentioned peak structure, some contrivances are necessary.

One of the methods for forming a target dose distribution is a scanning irradiation method. In order to perform the above-mentioned method, a function for arbitrarily deflecting an irradiation direction of a particle beam to a two-dimensional direction of XY which is perpendicular to a Z direction which is a travelling direction of a particle beam by using an electromagnet, etc. and a function for arbitrarily adjusting a position where a Bragg peak is formed, that is, an irradiation position to a Z direction by adjusting particle energy are necessary.

Generally, an accelerator which is a particle beam generating device has an energy adjusting function. Then, a plurality of irradiation positions (also called an irradiation spot) are set in a tumor, and by using the above-mentioned two functions, a particle beam is successively irradiated to each irradiation position. According to a scanning irradiation method, balance of an irradiation dose is determined in advance, each dose distribution which is formed when each irradiation position is irradiated is added up and accordingly, a target dose distribution is formed.

Then, in order to determine a dose of particle beam to be irradiated (a number of particle) to each irradiation position so as to make a three dimensional dose distribution which is added up to be a distribution which covers a tumor area for treating a tumor, it is necessary to obtain a dose distribution which is formed when a particle beam is irradiated to each irradiation position by simulation.

According to some conventional simulation devices which calculate a dose distribution, a beam is split to be a pulse function-shaped beamlet, an irradiation of each beamlet is overlapped so as to calculate a dose distribution in a patient's body. (For example, refer to Patent Document 1 and Patent Document 2)

Prior Art References

Patent Document

Patent Document 1 Japanese Patent Application Laid-Open JP 2011-217 880 A (FIG. 4, FIG. 5, etc.)
Patent Document 2 International publication WO00/015299

Disclosure of the Invention

Problems to be Solved by the Invention

According to a calculation method in which a dose distribution is calculated by splitting a beam into beamlets, in order to accurately calculate a three-dimensional dose distribution which is formed in a patient's body by irradiating one beam spot, it is necessary to generate many beamlets. Consequently, in a case where a calculation method in which a beam is split into beamlets is applied to a scanning irradiation method in which whole of an affected part is irradiated by a plurality of beam spots, there is a problem such that a great deal of calculation time is required.

In order to solve the above-mentioned problem, this invention has been made, and an object of this invention is to obtain a particle beam treatment-planning apparatus in which a three-dimensional dose distribution which is formed in a patient's body can be efficiently and accurately calculated by irradiating an irradiation spot successively while scanning an affected part with a particle beam.

Means for Solving the Problems

A particle beam treatment-planning apparatus;
which is provided in a particle beam therapy system in which by scanning a particle beam so as to shift and stay repeatedly using a scanning device for deflecting the particle beam to two-dimensional direction of XY which is perpendicular to a travelling direction of the particle beam, every stay of the particle beam, where a position of a depth corresponding to energy of the particle beam in an affected part of a patient which is an irradiation objective is set to be each irradiation position, each irradiation spot is formed, and by changing a position in a depth direction of the irradiation spot by changing energy of the particle beam, a three dimensional dose distribution is formed inside the patient including the affected part; comprises a calculation unit which obtains each irradiation dose of the particle beam which is irradiated to the each irradiation position,
wherein the calculation unit comprises a sub beam approximation unit in which the particle beam is approximated by a collection of a plurality of sub beams having a Gaussian distribution, individually, a sub beam dose distribution calculation unit in which a dose distribution of each sub beam of the plurality of sub beams which is formed in the patient by deflecting each sub beam by a scanning device and by adding up the dose distribution of each sub beam which is calculated, and each dose distribution which is formed inside the patient by the particle beam which is irradiated to each irradiation position is obtained, and an irradiation dose optimization unit in which each irradiation dose of the particle beam which is irradiated to the each irradiation position is obtained by an optimization calculation so as for the total dose distribution which is formed inside the patient and which is obtained by adding up each dose distribution which is formed inside the patient by the particle beam which is irradiated to the each irradiation position to be a target dose distribution which is set in a treatment plan.

Further a method for simulating a particle beam irradiation comprises a sub beam approximation step in which the particle beam is approximated by a collection of a plurality of sub beams having Gaussian distribution and a sub beam dose distribution calculation step in which by simulating the state in which each sub beam of the plurality of sub beams which is deflected by the scanning device and travelled, each sub beam dose distribution which is formed by the each sub beam inside the patient is calculated and the each sub beam dose distribution which is calculated is added up so as to obtain a dose distribution which is formed inside the patient by the particle beam..

Advantage of the Invention

According to this invention, a particle beam treatment-planning apparatus which can efficiently and accurately calculate a three dimensional dose distribution which is formed in a patient's body when a particle beam is irradiated according to a scanning irradiation method can be obtained.

Brief Description of the Drawings

FIG. 1    is a block diagram showing a configuration of a principal part of a particle beam treatment-planning apparatus according to Embodiment 1 of this invention.

FIG. 2    is a schematic diagram for describing a concept of a section of sub beam in a simulation of a particle beam treatment-planning apparatus according to Embodiment 1 of this invention.

FIG. 3    is a schematic diagram showing an example of approximation of sub beams in a simulation of a particle beam treatment-planning apparatus according to Embodiment 1 of this invention.

FIG. 4    is a schematic diagram for describing a concept of a simulation of a particle beam treatment-planning apparatus according to Embodiment 1 of this invention.

FIG. 5    is a flow chart showing a procedure of a simulation of a particle beam treatment-planning apparatus according to Embodiment 1 of this invention.

FIG. 6    is a schematic diagram showing another example of approximation of sub beams in a simulation of a particle beam treatment-planning apparatus according to Embodiment 1 of this invention.

FIG. 7    is a flow chart showing a procedure of a simulation of a particle beam treatment-planning apparatus according to Embodiment 2 of this invention.

FIG. 8    is a block diagram showing the configuration of a principal part of a particle beam treatment-planning apparatus according to Embodiment 2 of this invention.

FIG. 9    is a schematic diagram for describing a concept of a method for irradiating a particle beam to an affected part according to a scanning irradiation method.

FIG. 10    is a diagram for describing a dose distribution which is formed in a depth direction (Z direction) which is a travelling direction of a particle beam when irradiation is performed according to a scanning irradiation method.

FIG. 11    is a diagram for describing a dose distribution which is formed in one direction which is perpendicular to a travelling direction of a particle beam (X direction) when irradiation is performed according to a scanning irradiation method.

FIG. 12    is a schematic diagram for describing a concept of state in a case where a particle beam is irradiated to an affected part.

Modes for Carrying out the Invention

Embodiment 1

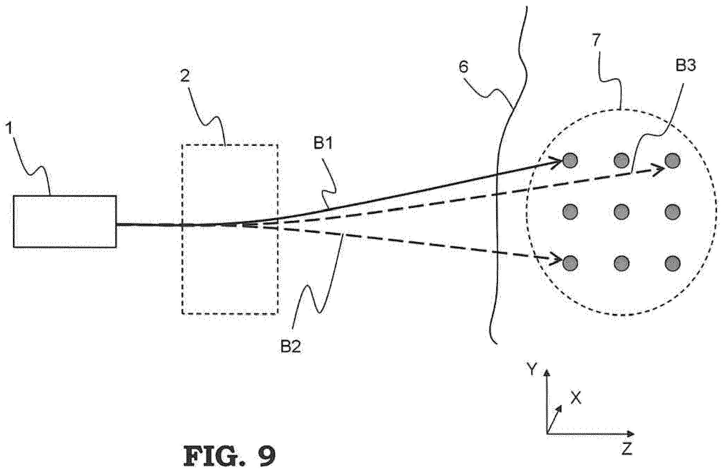

First, referring to FIGs. 9 to 12, a method for forming a dose distribution in a patient's body by irradiating a particle beam to an affected part of a patient such as a tumor according to a scanning irradiation method will be described. Regarding a particle beam therapy system using a scanning irradiation method, as shown in FIG. 9, a particle beam which is generated by a particle beam generating device 1 is deflected by a scanning device 2 to a lateral direction XY two dimension which is perpendicular to a travelling direction so as to scan an affected part of a patient.

A particle beam which is deflected has a thin pencil beam shape. In FIG. 9, a particle beam which is scanned is indicated by a particle beam B1, B2 and B3. Each particle beam is incident on a surface 6 of a body of a patient and stops at a predetermined depth position of an affected part 7. Irradiation positions of a particle beam are arranged so as to cover whole area of the affected part 7. Total of each dose distribution which is formed when a particle beam is irradiated to each irradiation position (distribution of a dose which is absorbed in a body) is a dose distribution which is formed in a patient's body.

In a treatment plan, it is planned such that the total dose distribution substantially conforms to an area of the affected part 7. In a treatment plan, an irradiation dose of a particle beam which is irradiated to each irradiation position is optimized so as to give a high dose which is necessary for treatment to an affected part and to give a dose as low as possible to normal tissue other than an affected part.

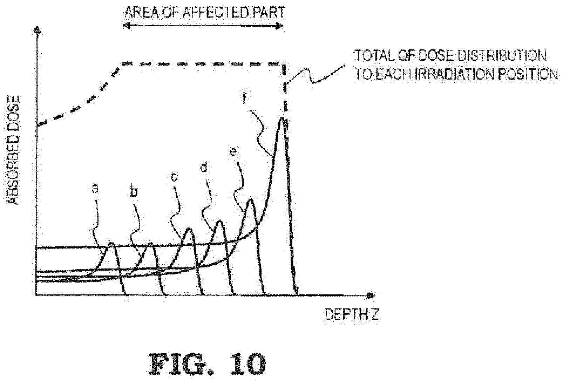

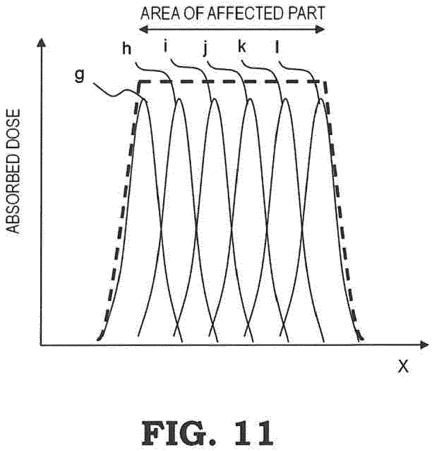

FIG. 10 and FIG. 11 show the state by every single dimension in which a three dimensional dose distribution is formed by a particle beam which scans an affected part. FIG. 10 shows an example of a dose distribution in a depth direction (Z direction) which is a travelling direction of a particle beam. Each curved line indicated by **a** to **f** shows an example of a dose distribution in a depth direction in a case where energy of a particle beam is different, individually.

As shown in FIG. 10, when energy of a particle beam is different, a peak position of an absorbed dose, that is, a position of a Bragg peak is different, therefore by changing energy of a particle beam, an irradiation position in a depth direction can be changed. In a case where each particle beam indicated by **a** to **f** whose energy is different is irradiated, the total of a dose distribution which is irradiated to each irradiation position, that is, a dose distribution which is formed by adding up each dose which is indicated by each curved line **a** to **f**, is a dose distribution which is irradiated to a patient.

In FIG. 10, a dose distribution which is irradiated to a patient is indicated by a broken line. As shown in FIG. 10, in general, irradiation is performed so as to make the total dose distribution to be a flat distribution in an area of an affected part of a patient.

On the other hand, FIG. 11 shows an example of a dose distribution in a depth of one direction which is perpendicular to a travelling direction of a particle beam, for example, at a Bragg peak position. Here, among scanning irradiation methods, an example of a spot scanning irradiation method in which irradiation is performed by shifting an irradiation position in a stepwise manner. In a spot scanning irradiation method, a particle beam is stayed at a certain irradiation position for performing irradiation, and after when an irradiation dose reaches a target dose, a particle beam is shifted to an adjacent irradiation position so as to stay for performing irradiation.

By repeating the above-mentioned process, a dose distribution is formed in whole area of an affected part in a lateral direction by shifting a particle beam in whole area of an affected part in a lateral direction. Each curved line indicated by **g** to **l** in FIG. 11 shows each dose distribution which is formed by each particle beam which stays at each irradiation position for performing irradiation, and a broken line shows the total dose distribution which is obtained by combining a dose distribution which is formed by a particle beam which is stayed at each irradiation position for performing irradiation. In FIG. 11, a distribution in an X-direction is shown, however, a distribution in a Y-direction is same.

As above mentioned, a dose distribution is formed in a three dimensional affected part by changing energy of a particle beam in a depth direction and by shifting a particle beam by a scanning device 2 in a lateral direction so as to irradiate a particle beam to each irradiation position of an affected part. Regarding a particle beam treatment, in a treatment-planning apparatus, each parameter of a particle beam to be irradiated is determined so as for a dose distribution to be a target dose distribution.

In order to determine each parameter in a treatment-planning apparatus, it is necessary to obtain an absorbed dose which is absorbed in an affected part by calculation, that is, it is necessary to perform a simulation. It is necessary to perform a simulation accurately as much as possible. This invention is made so as to provide a treatment-planning apparatus which can perform a simulation accurately and effectively as much as possible.

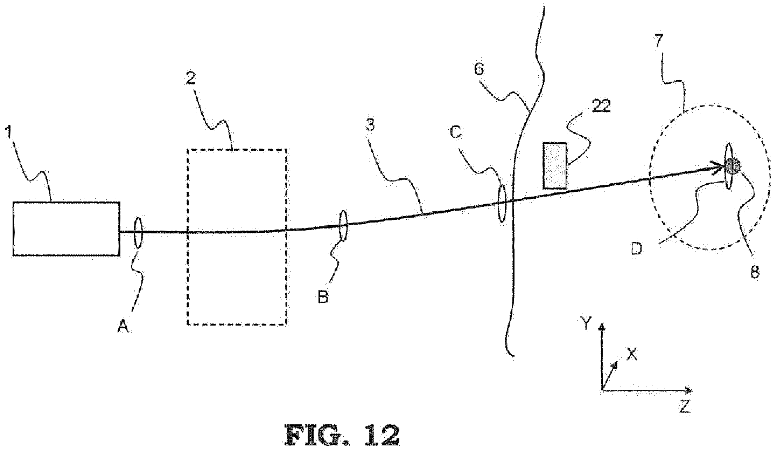

FIG. 12 conceptually shows the state in a case where a particle beam is actually irradiated to an affected part 7. FIG. 12 shows the state in which a sectional size of a particle beam 3 which is scanned changes toward an irradiation position 8, A, B, C and D. In FIG. 12, a part of the particle beam 3 passes in the vicinity of a bone area 22 before the particle beam 3 reaches the affected part 7. In a case where a particle beam is treated as one beam, the state in which a part of a particle beam passes through a different tissue can not be accurately simulated.

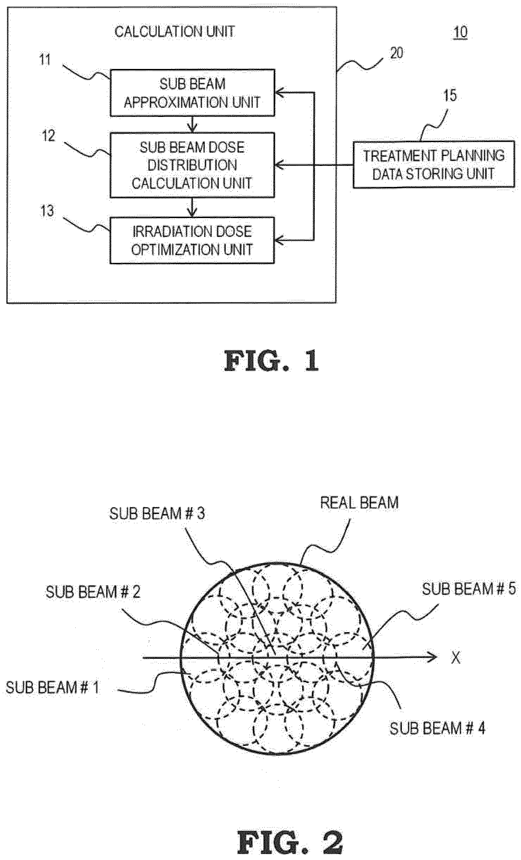

Then, a particle beam which is a pencil beam is approximated by a collection of a plurality of sub beams having Gaussian distribution, individually. FIG. 1 is a block diagram showing the configuration of a principal part of a particle beam treatment-planning apparatus according to Embodiment 1 of this invention.

FIG. 2 to FIG. 4 are schematic diagrams for describing a concept of a simulation of a particle beam treatment-planning apparatus according to Embodiment 1 of this invention. FIG. 2 is a schematic diagram showing a section of a particle beam and a solid line indicates an outline of a particle beam which is a pencil beam (hereinafter will be referred as a real beam).

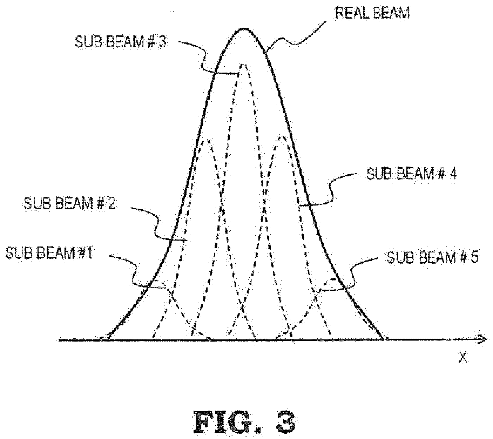

The real beam is approximated by a collection of a plurality of sub beams indicated by broken lines. An intensity distribution of a sub beam #1 to #5 which is arranged on an X-axis is shown in FIG. 3. In FIG. 3, an intensity distribution of a real beam indicated by a solid line can be approximated by resolving into a collection of a sub beam #1 to #5 whose section size is smaller than that of a real beam and which has an intensity distribution of Gaussian distribution, individually.

A distribution F (x, y) of a real beam is approximated by N sub beams $f(x-x_i, y-y_i)$ (i = 1 to N). When f (a, b) is a function of a Gaussian distribution, $f(x-x_i, y-y_i)$ is a function of a Gaussian distribution centering a position $(x_i, y_i)$. That is, $f(x-x_i, y-y_i)$ represents ith sub beam, that is, a distribution of sub beam #i. As shown in FIG. 3, by combining sub beams having different peak strength, a distribution of a real beam is approximated. That is, by giving weight Wi to a sub beam #i which is a function of a Gaussian distribution and combining all sub beams, a collection of a sub beam is determined so as to be

$$F(x, y) \cong \sum Wi*f(x-x_i, y-y_i) \qquad ...(1)$$

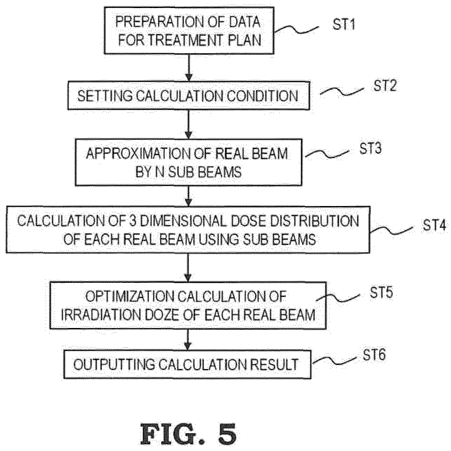

According to a particle beam treatment plan of this invention, each dose distribution which is formed in a patient's body by a particle beam which is irradiated to each irradiation position is simulated by using each sub beam in a right side of a formula (1). In calculating a three dimensional dose distribution, simulation is performed according to a flow shown in FIG. 5. First, a patient's three dimensional CT image which is necessary for a treatment plan and an irradiation parameter which is necessary for other irradiation such as an irradiation direction are prepared (Step ST1).

Next, a calculation condition such as a scanning pitch, an optimization calculation condition, etc. are set (Step ST2). Then, a real beam in a section is approximated by using N sub beams which have a Gaussian distribution having smaller sectional size. A center position of each sub beam (xi, yi), and its weight coefficient Wi (i = 1, 2, 3, ....N) are obtained by an optimization calculation, a collection of a sub beam is determined so as for the combined distribution shape to approximately reproduce a distribution shape of a real beam 3, that is, to satisfy the formula (1) (Step ST3).

The approximation certifies the accuracy of a three dimensional dose distribution of a real beam based on a dose distribution calculation of a sub beam which will be described later, and is an important character of this invention. Especially, as shown in FIG. 3, the points, an intensity distribution of a sub beam has a Gaussian distribution, and a distribution width of the Gaussian distribution is smaller than that of a real beam, are important.

Then, calculation of a three dimensional dose distribution of the particle beam 3 in a patient's body which is scanned is performed as follows. A three dimensional dose distribution in a patient's body is obtained by calculation (weighted convolution calculation) in which each calculation result of a three dimensional dose distribution which is formed in a body by each sub beam is multiplied by each weight coefficient $W_i$ (i = 1, 2, 3 ... ... N) and added up.

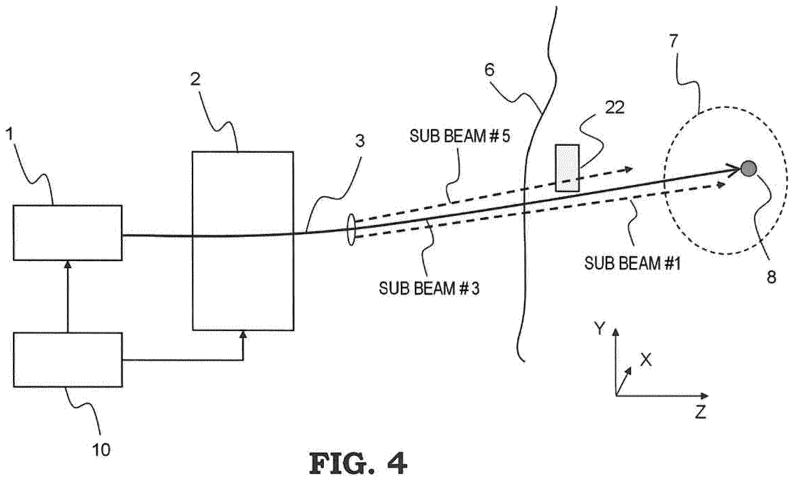

For example, as shown in FIG. 4, a three dimensional dose distribution is formed so as for sub beam #5 which is a fifth sub beam of the particle beam 3 which is scanned to pass through the bone area 22 and stop at a position between the bone area 22 and an irradiation position 8. On the other hand, a sub beam #3 passes through an area other than the bone area 22, stops at the irradiation position 8, and a three dimensional dose distribution is formed in the trajectory.

Then, in the same way, regarding sub beam #1, a predetermined three dimensional dose distribution is formed along other trajectory other than the bone are 22 toward the irradiation position 8.

Regarding all sub beams, each three dimensional dose distribution is calculated, a result is multiplied by each

weight coefficient $W_i$ and the total is a three dimensional dose distribution of a real beam (Step ST4). In Step ST4, regarding each real beam which is irradiated to each irradiation spot, the above-mentioned calculation is performed. In Step ST4, for example, each three dimensional dose distribution in a case where each irradiation spot is irradiated with unit irradiation dose is calculated.

By using each three dimensional dose distribution in a case where each irradiation spot is irradiated with unit irradiation dose, when an irradiation dose of a particle beam to be irradiated to each irradiation spot is set to be $MU_j$ ($j = 1, 2, 3 ......M$:M indicates a number of spots in spot scanning irradiation), by adding up a three dimensional dose distribution in a case where each irradiation spot is irradiated, a three dimensional dose of whole of an affected part can be calculated. Here, each $MU_j$ is determined by optimization calculation. Optimization calculation is performed by using an optimization algorithm which is input together with other parameter in Step ST1 and Step ST2 and by determining each $MU_j$ so as for a dose distribution of whole of an affected part to be a dose distribution which is close to a target dose distribution (Step ST5). A list of irradiation dose $MU_j$ ($j = 1, 2, 3 ... ...M$) which is determined, a dose distribution in a patient's body, etc. are output (Step ST6). In actual particle beam treatment, based on an irradiation dose of a particle beam which is output at each spot $MU_j$ ($j = 1, 2, 3 ... ...M$), a particle beam is irradiated to a patient.

As shown in a block diagram of FIG. 1, the above-mentioned flow is performed in a calculation unit 20 in a particle beam treatment-planning apparatus 10. An irradiation parameter which is prepared in Step ST1 and a calculation condition which is set in Step ST2 are stored in a treatment planning data storing unit 15. Step ST3 is performed in a sub beam approximation unit 11, Step ST4 is performed in a sub beam dose distribution calculation unit 12 and Step ST5 is performed in an irradiation dose optimization unit 13, respectively.

As above mentioned, in order to form a predetermined three dimensional distribution in an affected area by irradiating a particle beam according to a scanning irradiation method, an intensity distribution of a particle beam is approximated to be a Gauss shape, and further, the Gauss shape is approximated by a collection of a plurality of sub beams having the Gaussian distribution. By simulating the state in which each sub beam of the plurality of sub beams are deflected by a scanning device and travelled, a three dimensional dose which is formed in a patient's body is calculated. Consequently, when a sub beam passes in the vicinity of the bone area 22 which exists in a patient's body, a three dimensional dose distribution of a particle beam can be calculated accurately taking account of an effect in the bone area. Further, a position of each sub beam ($x_i$, $y_i$) and its weight coefficient $W_i$ ($i = 1, 2, 3 ... ... N$) is obtained by optimization calculation and a distribution of a real beam is approximately reproduced by the combined shape. Consequently, as approximation of a real beam, the accuracy can be certified, and a three dimensional dose distribution which is formed by a real beam which is scanned can be calculated with high accuracy.

Regarding the approximation, the points, that is, an intensity distribution of a sub beam is a Gaussian distribution and a distribution width of the Gaussian distribution is smaller than a distribution width of a real beam, are especially important. According to the above mentioned points, an intensity distribution of a real beam can be approximated with high accuracy and high calculation accuracy can be obtained.

In this invention, the state in which each sub beam of a plurality of sub beams is deflected and travelled by a scanning device is simulated. That is, by calculating a trajectory of each sub beam separately, a simulation can be performed so as for each sub beam to travel different positions, therefore, the phenomena in which as a real beam travels toward travelling direction, a size of a beam becomes larger can be accurately reproduced. Especially in a case where a medium is non-uniform, the phenomena, that is, the process that a beam size becomes larger along with travelling of beam is changed depending on a position, can be accurately reproduced.

Further, in general, it is well known such that a distribution in an XY direction of a particle beam which travels in a medium can be accurately approximated by a Gaussian distribution, (For example, a formula of Highland, (Refer to V.L. Highland, "Some practical remarks on multiple scattering", Nucl. Instrum & Method b,74,497,1993)), by using a Gaussian distribution as a distribution of a sub beam, a three dimensional dose distribution which is formed in a patient's body can be calculated more accurately. From the view of the above-mentioned, a particle beam treatment-planning apparatus which is highly accurate can be obtained.

In the above-mentioned, a case in which an intensity distribution of a real beam is approximated to a Gaussian distribution is described, however, an actual real beam distribution is not necessarily limited to be a Gaussian distribution. Especially, in a case where a particle beam is a carbon ion beam, in the vicinity of an axis of a real beam, an intensity distribution of a beam can be approximated by a Gaussian distribution, however at a position which is far away from an axis, a distribution which is largely trailed to both ends, which is called "large angle component" exits, and due to this influence, in a method in which a real beam is approximated by a Gaussian distribution, it is possible such that a final dose distribution can not be accurately calculated.

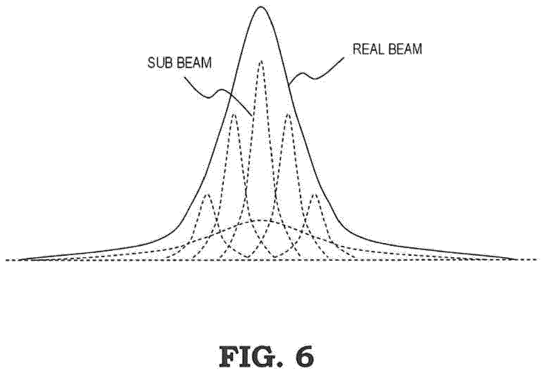

Even in the above-mentioned case, as shown in FIG. 6, by introducing a sub beam having more than one Gaussian distributions corresponding to a large angle component, a distribution of trail of a real beam can be reproduced, and according to the above-mentioned method, a dose distribution which is formed in a patient's body can be calculated.

Embodiment 2

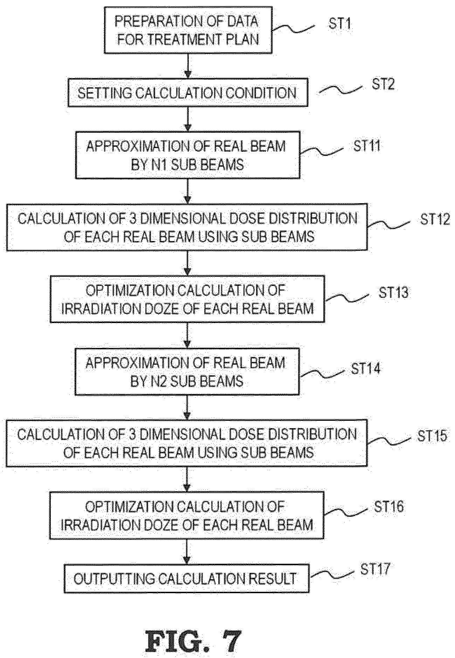

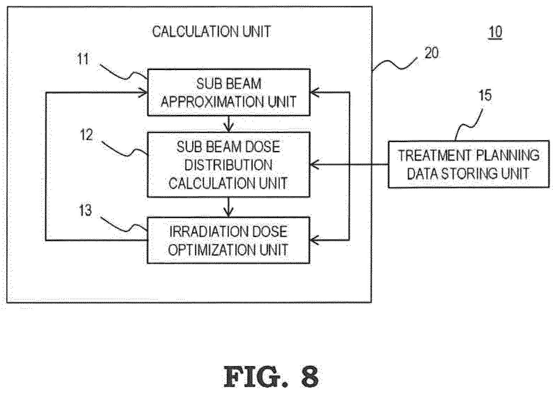

FIG. 7 is a flow chart showing a procedure of a simulation of a particle beam treatment-planning apparatus according to Embodiment 2 of this invention and FIG. 8 is a block diagram showing the configuration of a particle beam treatment-planning apparatus according to Embodiment 2 of this invention. Hereinafter, Embodiment 2 of this invention will be described based on FIG. 7 and FIG. 8.

First, a patient's three dimensional CT image which is necessary for a treatment plan and other parameter such as an irradiation direction, etc. are prepared (Step ST1). Next, calculation conditions including a scanning pitch, a calculation condition for optimization calculation, etc. are prepared (Step ST2). Next, when the number of sub beams is N1 (N1 = 1, 3, 5, 7, 9...), a particle beam 3 (a real beam) shown in FIG. 1 is approximated by a sub beam having a distribution of a Gauss shape whose section size is smaller than that of a real beam in a section of a real beam as shown in FIG. 2 (however, in a case where N1 = 1, a section size is same as that of a real beam) (Step ST11). That is, a center position of each sub beam ($x_i$, $y_j$) and its weight coefficient $W_i$ (i = 1, 2, 3 ... ...N1) are obtained by optimization calculation, a collection of sub beams is determined so as for the combined shape to approximately reproduce a distribution of a real beam, that is, so as to satisfy formula (1). A collection of N1 sub beams is set to be a first a collection of sub beams.

Then, calculation of a three dimensional dose distribution of the particle beam 3 which is scanned in a patient's body will be performed as follows. A three dimensional dose distribution in a patient's body can be obtained by multiplying each calculation result of the three dimensional dose distribution of each sub beam which is formed in a body by each its weight coefficient $W_i$ (i = , 2, 3, ... ...N) and adding up (weighted convolution calculation).

For example, as shown in FIG. 4, a three dimensional dose is formed so as for a fifth sub beam #5 of the particle beam 3 which is scanned to pass through a bone area 22 and stop between the bone area 22 and an irradiation position 8. On the other hand, a three dimensional dose distribution is formed in the trajectory so as for a sub beam #3 to pass through an area other than the bone area 22 and stop at the irradiation position 8. Then, in the same way, regarding a sub beam #1, a predetermined three dimensional dose distribution is formed along other trajectory other than the bone area 22 toward the irradiation position 8.

As above mentioned, regarding all sub beams of a first collection of sub beams, each three dimensional dose distribution is calculated, the result is multiplied by each weight coefficient $W_i$, and the total is a three dimensional dose distribution of a real beam (Step ST12). In Step ST12, regarding each real beam which is irradiated to each spot, the above-mentioned calculation is performed. In Step ST12, for example, each three dimensional dose distribution in a case where each irradiation spot is irradiated with a unit irradiation dose is calculated.

By using each three dimensional dose distribution in a case where each irradiation spot is irradiated with an unit irradiation dose and by adding up a three dimensional dose distribution in a case where each irradiation spot is irradiated when an irradiation dose of a particle beam to be irradiated to each irradiation spot is set to be $MU_j$ (j = 1, 2, 3, ...... M: M is a number of spots in a spot scanning irradiation), a three dimensional dose distribution of whole of an affected part can be calculated. Then, each $MU_j$ is determined by optimization calculation. Optimization calculation is performed by using an optimization algorithm which is input with other parameter in Step ST1 and Step ST2 and determining each $MU_j$ so as for a dose distribution in a whole of an affected part to be close to a target dose distribution (Step ST13).

Thus far, an operation flow of a particle beam treatment-planning apparatus is basically same as that shown in Embodiment 1. In Embodiment 2, next, the number of sub beams is set to be N2 which is larger than N1, by a second collection of sub beams whose number is N2 which is larger than the number of a first collection of sub beams whose number is N1, a real beam is approximated (Step ST14). Then, in the same way as that of Step ST12, regarding the second collection of sub beams, each three dimensional dose distribution is calculated, the result is multiplied by each weight coefficient $W_i$ (i = 1, 2, 3, ... ... N2) and the result which is added up is set to be a three dimensional dose distribution of the real beam 3.

Regarding each real beam to be irradiated to each spot, the above-mentioned calculation is performed (Step ST15). Then, in Step ST16, in the same way as that of Step ST13, an irradiation dose of each real beam is calculated by optimization calculation. In Step ST16, as an initial irradiation dose of each real beam of optimization calculation, $MU1_j$ (j = 1, 2, 3, ......M) which is obtained in ST13 is used. An irradiation dose which is obtained by optimization calculation is set to be $MU2_j$ (j = 1, 2, 3... ...M), a list of $MU2_j$ and a dose distribution in whole of an affected part is output (Step ST17). In actual particle beam treatment, based on an irradiation dose of a particle beam $MU2_j$ (j = 1, 2, 3 ... ...M) at each spot which is output, a particle beam is irradiated to a patient.

As shown in FIG. 8, the above-mentioned flow is performed in a calculation unit 20 in a particle beam treatment-planning apparatus 10. An irradiation parameter which is prepared in Step ST1, a calculation condition which is set in Step ST2, etc. are stored in a treatment planning data storage unit 15. Step ST11 and Step ST14 are performed in a sub beam approximation unit 11, Step ST12 and Step ST15 are performed in a sub beam dose distribution calculation unit 12, and Step ST13 and Step ST16 are performed in an irradiation dose optimization unit 13, respectively.

As above mentioned, in Embodiment 2, first, the number of sub beams is set to be N1 which is a relatively small number, and roughly, an irradiation dose of each real beam $MU1_j$ (j = 1, 2, 3 ... ...) is calculated. Then, the number of sub beams is set to be N2 which is a relatively large number, by calculating a three dimensional dose distribution in an affected part accurately, finally, an irradiation dose $MU2_j$ of each particle beam with high accuracy can be calculated. Here, N1 is set to be a relatively small number such as 1 or 3, and N2 is set to be a relatively large number such as 21, for example. N1 which is the number of a first collection of sub beams is small.

The time which is required for calculating a three dimensional dose distribution is substantially proportional to N1, therefore, the first step ST11 to ST13 can be performed at high speed. Once an irradiation dose of a particle beam which is approximately irradiated to each irradiation position $MU1_j$ (j = 1, 2, 3 ... ...M) is obtained, in Step ST14, the number of sub beams of N2 is set to be larger than that of N1 and a second collection of sub beams is determined, in Step ST16, by performing optimization calculation in which an irradiation dose $MU1_j$ (j = 1, 2, 3 ... ...M) is set to be an initial value, converged $MU2_j$ (j = 1, 2, 3... ...M) can be obtained by the smaller number of times.

By doing the above mentioned, a compromise between a calculation accuracy and a calculation speed can be reached, more effectively and in a comparatively short time, an irradiation dose of a particle beam $MU2_j$ (j = 1, 2, 3 ... ...M) can be obtained. Consequently, according to this invention, a particle beam treatment plan in which calculation speed is fast and an calculation accuracy is high can be obtained.

| | |
|---|---|
| 2 | scanning device |
| 3 | real beam |
| 10 | particle beam treatment-planning apparatus |
| 11 | sub beam approximation unit |
| 12 | sub beam dose distribution calculation unit |
| 13 | irradiation dose optimization unit |
| 20 | calculation unit |

**Claims**

1. A particle beam treatment-planning apparatus;
   which is provided in a particle beam therapy system in which by scanning a particle beam so as to shift and stay repeatedly using a scanning device for deflecting the particle beam to two-dimensional direction of XY which is perpendicular to a travelling direction of the particle beam,
   every stay of the particle beam, where a position of a depth corresponding to energy of the particle beam in an affected part of a patient which is an irradiation objective is set to be each irradiation position, each irradiation spot is formed, and
   by changing a position in a depth direction of the irradiation spot by changing energy of the particle beam, a three dimensional dose distribution is formed inside the patient including the affected part;
   comprising a calculation unit which obtains each irradiation dose of the particle beam which is irradiated to the each irradiation position,
   wherein the calculation unit comprises

   - a sub beam approximation unit in which the particle beam is approximated by a collection of a plurality of sub beams having a Gaussian distribution, individually,
   - a sub beam dose distribution calculation unit in which a dose distribution of each sub beam of the plurality of sub beams which is formed in the patient by deflecting each sub beam by a scanning device and by adding up the dose distribution of each sub beam which is calculated, and each dose distribution which is formed inside the patient by the particle beam which is irradiated to each irradiation position is obtained, and
   - an irradiation dose optimization unit in which each irradiation dose of the particle beam which is irradiated to the each irradiation position is obtained by an optimization calculation so as for the total dose distribution which is formed inside the patient and which is obtained by adding up each dose distribution which is formed inside the patient by the particle beam which is irradiated to the each irradiation position to be a target dose distribution which is set in a treatment plan.

2. The particle beam treatment-planning apparatus of claim 1,
   wherein in the sub beam approximation unit, each of the particle beam is separately approximated by two collections of sub beams, a first collection of sub beams whose number is relatively small and a second collection of sub beams whose number is relatively large,

in the irradiation dose optimization unit, each irradiation dose of the particle beam, which is irradiated to the each irradiation position, which is a result which is obtained by using the first collection of sub beams is set to be an initial value, by using the second collection of sub beams, each irradiation dose of the particle beam which is irradiated to the each irradiation position is obtained.

3. A method for simulating a particle beam irradiation, by which a dose distribution, which is formed inside a patient when a particle beam having a pencil beam shape is deflected by a scanning device to be irradiated to an affected part of the patient, is simulated, comprising a sub beam approximation step in which the particle beam is approximated by a collection of a plurality of sub beams having Gaussian distribution, individually, and

a sub beam dose distribution calculation step in which by simulating the state in which each sub beam of the plurality of sub beams which is deflected by the scanning device and travelled, each sub beam dose distribution which is formed by the each sub beam inside the patient is calculated and the each sub beam dose distribution which is calculated is added up so as to obtain a dose distribution which is formed inside the patient by the particle beam.

4. A method for simulating a particle beam irradiation;

by which a dose distribution in an affected part of a patient which is an irradiation objective in a particle beam therapy system is obtained by simulation,

in the particle beam therapy system, by scanning a particle beam so as to shift and stay repeatedly using a scanning device for deflecting the particle beam to two-dimensional direction of XY which is perpendicular to a travelling direction of the particle beam,

every stay of the particle beam, where a position of a depth corresponding to energy of the particle beam in the affected part is set to be each irradiation position, each irradiation spot is formed, and

by changing a position in a depth direction of the irradiation spot by changing energy of the particle beam, a three dimensional dose distribution is formed inside the patient including the affected part;

comprising a sub beam approximation step in which the particle beam is approximated by a collection of a plurality of sub beams having Gaussian distribution, individually, and

a sub beam dose distribution calculation step in which by simulating the state in which each sub beam of the plurality of sub beams which is deflected by the scanning device and travelled, each sub beam dose distribution which is formed by the each sub beam inside the patient is calculated and the each sub beam dose distribution which is calculated is added up so as to obtain each dose distribution which is formed inside the patient by the particle beam to be irradiated to the each irradiation position.

5. The method for simulating a particle beam irradiation of claim 4, further comprising

an irradiation optimization step in which each irradiation dose of the particle beam which is irradiated to the each irradiation position is obtained by optimization calculation so as for all dose distributions which are formed inside the patient which are obtained by adding up the each dose distribution which is obtained in the sub beam dose distribution calculation step to be a target dose distribution which is set in a treatment plan.

6. The method for simulating a particle beam irradiation of claim 5,

wherein in the sub beam approximation step, the particle beam is separately approximated by two collections, a first collection of sub beams whose number is relatively small and a second collection of sub beams whose number is relatively large,

in the irradiation dose optimization step, each irradiation dose of the particle beam, which is irradiated to the each irradiation position, which is a result which is obtained by using the first collection of sub beams, is set to be an initial value for the optimization calculation, by using the second collection of sub beams, each irradiation dose of the particle beam which is irradiated to the each irradiation position is obtained.

CALCULATION UNIT

11

SUB BEAM
APPROXIMATION UNIT

12

SUB BEAM DOSE
DISTRIBUTION
CALCULATION UNIT

13

IRRADIATION DOSE
OPTIMIZATION UNIT

20

10

15

TREATMENT PLANNING
DATA STORING UNIT

# FIG. 1

SUB BEAM # 3

REAL BEAM

SUB BEAM # 2

SUB BEAM # 5

X

SUB BEAM # 1

SUB BEAM # 4

# FIG. 2

REAL BEAM

SUB BEAM # 3

SUB BEAM # 4

SUB BEAM # 2

SUB BEAM #1

SUB BEAM # 5

X

**FIG. 3**

FIG. 4

FIG. 5

SUB BEAM

REAL BEAM

FIG. 6

PREPARATION OF DATA
FOR TREATMENT PLAN — ST1

SETTING CALCULATION CONDITION — ST2

APPROXIMATION OF REAL BEAM
BY N1 SUB BEAMS — ST11

CALCULATION OF 3 DIMENSIONAL DOSE DISTRIBUTION
OF EACH REAL BEAM USING SUB BEAMS — ST12

OPTIMIZATION CALCULATION OF
IRRADIATION DOZE OF EACH REAL BEAM — ST13

APPROXIMATION OF REAL BEAM
BY N2 SUB BEAMS — ST14

CALCULATION OF 3 DIMENSIONAL DOSE DISTRIBUTION
OF EACH REAL BEAM USING SUB BEAMS — ST15

OPTIMIZATION CALCULATION OF
IRRADIATION DOZE OF EACH REAL BEAM — ST16

OUTPUTTING CALCULATION RESULT — ST17

# FIG. 7

**FIG. 8**

**FIG. 9**

EP 3 130 375 A1

**FIG. 10**

FIG. 11

**FIG. 12**

## INTERNATIONAL SEARCH REPORT

| International application No. |
|---|
| PCT/JP2014/060089 |

**A. CLASSIFICATION OF SUBJECT MATTER**
*A61N5/10(2006.01)i*

According to International Patent Classification (IPC) or to both national classification and IPC

**B. FIELDS SEARCHED**

Minimum documentation searched (classification system followed by classification symbols)
A61N5/10

Documentation searched other than minimum documentation to the extent that such documents are included in the fields searched
Jitsuyo Shinan Koho          1922-1996    Jitsuyo Shinan Toroku Koho    1996-2014
Kokai Jitsuyo Shinan Koho    1971-2014    Toroku Jitsuyo Shinan Koho    1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2011-217880 A  (National Cancer Center),<br>04 November 2011 (04.11.2011),<br>paragraphs [0022] to [0025], [0041], [0048];<br>fig. 1, 5, 10 to 11<br>(Family: none) | 1,3-5<br>2,6 |
| A | JP 2012-80983 A  (Hitachi, Ltd.),<br>26 April 2012 (26.04.2012),<br>paragraph [0031]<br>(Family: none) | 1-6 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 May, 2014 (27.05.14) | 10 June, 2014 (10.06.14) |

| Name and mailing address of the ISA/<br>   Japanese Patent Office | Authorized officer |
|---|---|
| Facsimile No. | Telephone No. |

Form PCT/ISA/210 (second sheet) (July 2009)

**REFERENCES CITED IN THE DESCRIPTION**

**Patent documents cited in the description**

- JP 2011217880 A

- WO 00015299 A